# EUROPEAN PATENT APPLICATION

(11) **EP 3 495 351 A1**
(43) Date of publication of application: **12.06.2019**
(21) Application number: 17206219.2
(22) Date of filing: 08.12.2017
(51) Int. Cl.: C07D 231/14

(54) **OXIDATION OF A PYRAZOLYL KETONE COMPOUND TO THE CORRESPONDING CARBOXYLIC ACID**

(71) Applicant: SOLVAY SA, 1120 Brussels (BE)
(72) Inventor: MERCIER, Claude, 84150 JONQUIERES (FR)
(74) Representative: Mross, Stefan P.M.

(57) **Abstract**

This invention concerns a process for the manufacture of carboxylic acids or carboxylic acid derivatives and a process for the manufacture of agrochemically and pharmaceutically active compounds comprising the process for the manufacture of carboxylic acids or their derivatives. The process for the manufacture of carboxylic acids or carboxylic acid derivatives of formula (II) comprises a step of oxidizing a compound of formula (I) in the presence of at least one compound of formula (III) R⁶-C(O)O-OH.

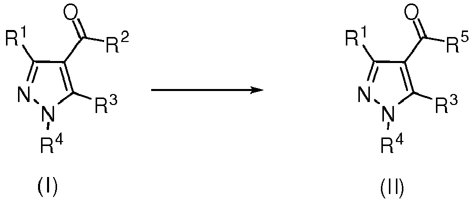

## Description

This invention concerns a process for the manufacture of carboxylic acids or carboxylic acid derivatives and a process for the manufacture of agrochemically and pharmaceutically active compounds comprising the process for the manufacture of carboxylic acids or their derivatives.

Carboxylic acid and their derivatives, in particular 3-halomethylpyrazol-4-yl carboxylic acids and esters, are valuable intermediates in the synthesis of agrochemical and pharmaceutical active ingredients. Agrochemical active ingredients which contain 3-halomethylpyrazol-4-yl building blocks are, for example, 2'-[1,1'-bicycloprop-2-yl]-3-(difluoromethyl)-1-methylpyrazole-4-carboxanilide (Sedaxane), as described, for example, in WO2006015866, 3-(difluoromethyl)-1-methyl-N-[2-(3',4',5'-trifluorophenyl)phenyl]pyrazole-4-carboxamide (Fluxapyroxad), as described, for example, in WO2006087343, N-(3',4'-Dichloro-5-fluorobiphenyl-2-yl)-3-(difluoromethyl)-1-methylpyrazole-4-carboxamide (Bixafen), as described, for example, in WO2003070705, 3-(Difluoromethyl)-1-methyl-N-[1,2,3,4-tetrahydro-9-(1-methylethyl)-1,4-methanonaphthalen-5-yl]-1H-pyrazole-4-carboxamide (Isopyrazam), as described, for example, in WO2004035589, (RS)-N-[9-(Dichloromethylen)-1,2,3,4-tetrahydro-1,4-methanonaphthalin-5-yl]-3-(difluoromethyl)-1-methyl-1H-pyrazole-4-carboxamide (Benzovindiflupyr), as described, for example, in WO07048556. Generally, 3-halomethylpyrazol-4-yl carboxylic acids, often obtained by hydrolysis of their esters, are converted into the carboxamides, for example after conversion into the 3-halomethylpyrazol-4-yl carboxylic acid halide. Other conversions, wherein the carboxamide is generated directly from the ester or acid, have also been described, such as in WO2012055864 and WO 2007/031323. All foregoing cited patent applications are hereby incorporated for all purposes.

WO2016/152886 discloses oxidation of pyrazolyl ketone derivatives with hypohalite solutions. When hypohalites are used for oxidation of the keto function, a large amount, at least three equivalents, of hypohalite is necessary to convert the keto group into a carboxylate salt. This results in a large volume of salt waste per mole carboxylate produced, which is often also difficult to treat because of its organic impurities. As hypohalite solutions are often restricted in their upper concentration limit due to stability concerns, the waste volume is even higher per mole carboxylate produced.

It has been found that the process according to the present invention allows for the manufacture of a carboxylic acid or its derivative efficiently and environmentally friendly. The process shows good yields, low waste and can be processed on a large scale.

The invention thus concerns a process for the manufacture of a compound of formula (II) comprising a step of oxidizing a compound of formula (I) in the presence of at least one compound of formula (III) R⁶-C(O)O-OH 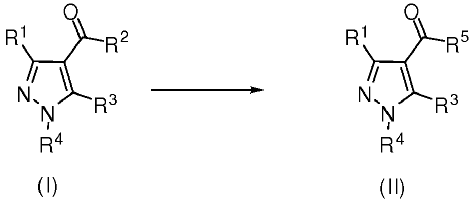 wherein R¹, R², R³, R⁴ and R⁵ will be defined in the description. The invention further concerns a process as shown above wherein the process for the manufacture of a compound of formula (II) further comprises a step of reacting a compound of formula (IV) with a compound selected from the group consisting of compound (V), (VI) and (VII) to obtain the compound of formula (I) 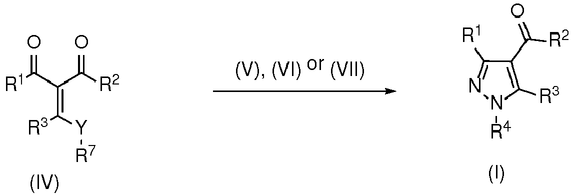 wherein Y, R⁷ and compounds (V), (VI) and (VII) are defined in the description. The invention also concerns a process for the manufacture of an agrochemical or pharmaceutical compound, preferably a carboxamide SDHI (succinate dehydrogenase inhibitor) fungicide, which comprises the processes as shown above for the manufacture of compound (II).

In the present invention, designations in singular are in intended to include the plural; for example, "a solvent" is intended to denote also "more than one solvent" or "a plurality of solvents".

All aspects and embodiments of the present invention are combinable.

In the context of the present invention, the term "comprising" is intended to include the meaning of "consisting of".

When a double bond is depicted in a particular E/Z geometry, this is intended to also denote the other geometric form as well as mixtures thereof.

In a first aspect, the invention concerns a process for the manufacture of a compound of formula (II) comprising a step of oxidizing a compound of formula (I) in the presence of at least one compound of formula (III) R⁶-C(O)O-OH 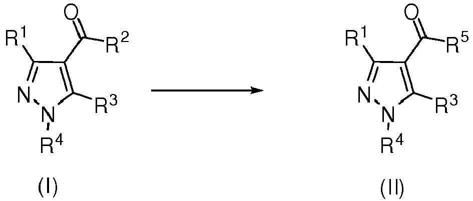 wherein
R¹ is selected from the group consisting of C₁-C₄-alkyl groups which is substituted by at least one halogen atom independently selected from the group consisting of F, Cl and Br or by a CF₃ group,
R² is selected from the group consisting of an optionally substituted C₁-C₈ alkyl group, an optionally substituted C₃-C₈ cycloalkyl group, an optionally substituted aryl group and an optionally substituted heteroaryl group,
R³ is selected from the group consisting of H, X, COOR, OR, SR, C(O)NR₂, wherein R is selected from the group consisting of hydrogen, a C₁-C₁₂-alkyl, CN, C₂-C₆ alkenyl, aryl, C₃-C₈ cycloalkyl, aralkyl or heteroaryl group, each of which is optionally substituted, with the proviso that both R in C(O)NR₂ may be the same or different, wherein X is a halogen atom and is selected from the group consisting of F, Cl, Br and I, and R is independently selected from the group consisting of hydrogen or a C₁-C₁₂-alkyl, C₂-C₆ alkenyl, aryl, cycloalkyl, aralkyl, heteroaryl,
R⁴ is selected from the group consisting of H, C₁-C₁₂-alkyl, C₂-C₆ alkenyl, C₃-C₈ cycloalkyl, aryl, heteroaryl or aralkyl group, each of which is optionally substituted,
R⁵ is an -OH group, and
R⁶ is selected from the group consisting of an optionally substituted C₁-C₈ alkyl group, an optionally substituted C₃-C₈ cycloalkyl group, an optionally substituted aryl group and an optionally substituted heteroaryl group.

In the context of the present invention, the term "C₁-C₁₂-alkyl groups" is intended to denote straight or branched alkyl groups having one to twelve carbon atoms. Additionally to the groups comprised in the "C₁-C₈ alkyl group", and "C₁ to C₄ group as defined below, the term "C₁-C₁₂-alkyl groups" comprises, for example, n-nonyl and its isomers, n-decyl and its isomers, n-undecyl and its isomers and n-dodecyl and its isomers. The term "C₁-C₈ alkyl group" intends to denote straight or branched alkyl groups having one to eight carbon atoms. This group comprises in particular methyl, ethyl, n-propyl, isopropyl, n-, iso-, sec-and t-butyl, n-pentyl and its isomers, n-hexyl and its isomers, 1,3-dimethylbutyl, 3,3-dimethylbutyl, n-heptyl and its isomers and n-octyl and its isomers. The term "C₁-C₄-alkyl groups" is intended to denote straight or branched alkyl groups having one to four carbon atoms. This group comprises in particular methyl, ethyl, n-propyl, isopropyl, n-, iso-, sec- and t-butyl. Often, the C₁-C₄ alkyl groups are the most preferred groups, with methyl being the most preferred group.

The term "C₃-C₈ cycloalkyl group" intends to denote mono-, bi- or tricyclic hydrocarbon groups comprising 3 to 10 carbon atoms, especially 3 to 6 carbon atoms. Examples of monocyclic groups include cyclopropyl, cyclobutyl, cyclopentyl, cyclohexyl, cycloheptyl or cyclooctyl. Examples of bicyclic groups include bicyclo[2.2.1]heptyl, bicyclo[3.1.1]heptyl, bicyclo[2.2.2]octyl and bicyclo[3.2.1]octyl. Examples of tricyclic groups are adamantyl and homoadamantyl.

The term "aryl group" intends to denote C₅-C₁₈ monocyclic and polycyclic aromatic hydrocarbons with 5 to 18 carbon atoms in the cyclic system. Specifically, this definition comprises, for example, the meanings cyclopentadienyl, phenyl, cycloheptatrienyl, cyclooctatetraenyl, naphthyl and anthracenyl.

The term "heteroaryl group" intends to denote C₅-C₁₈ monocyclic and polycyclic aromatic hydrocarbons with 5 to 18 carbon atoms in the cyclic system, wherein one or more methine (-C=) and/or vinylene (-CH=CH-) groups are replaced by trivalent or divalent heteroatoms, in particular nitrogen, oxygen and/or sulphur, respectively, in such a way as to maintain the continuous π-electron system characteristic of aromatic systems. Specifically, this definition comprises, for example, the meanings 2-furyl, 3-furyl, 2-thienyl, 3-thienyl, 2-pyrrolyl, 3-pyrrolyl, 3-isoxazolyl, 4-isoxazolyl, 5-isoxazolyl, 3-isothiazolyl, 4-isothiazolyl, 5-isothiazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl; 1-pyrrolyl, 1-pyrazolyl, 1,2,4-triazol-1-yl, 1-imidazolyl, 1,2,3-triazol-1-yl, 1,3,4-triazol-1-yl; 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl.

The term "C₂-C₆ alkenyl" intends to denote a group comprising a carbon chain and at least one double bond. Alkenyl group are, for example, ethenyl, propenyl, butenyl, pentenyl or hexenyl.

The term "aralkyl" intends to denote alkyl groups which are substituted by aryl groups, which have a C₁-C₈-alkylene chain and which may be substituted in the aryl skeleton or the alkylene chain by one or more heteroatoms selected from the group consisting of O, N, P and S.

Where indicated, a C₁-C₄ alkyl group, C₁-C₈ alkyl group, C₁-C₁₂ alkyl group, aryl group, heteroaryl group, C₁-C₁₂-alkyl group, C₂-C₆ alkenyl group, aryl group, aralkyl group or heteroaryl group each can be optionally substituted by one or more substituents of the group consisting of R', -X', -OR', -SR', -NR'₂, -SiR'₃, -COOR', -(C-O)R', -CN and -CONR'₂, wherein R' is selected independently, from the group consisting of hydrogen or C₁-C₁₂-alkyl group and X' is selected from the group consisting of F, Cl, Br, or I.

R¹ is selected from the group consisting of C₁-C₄-alkyl groups, which are substituted by at least one halogen atom independently selected from the group consisting of F, Cl and Br or by a CF₃ group. Preferably, R₁ is selected from the group consisting of CF₂H, CF₃, CClF₂, CCl₂H and CCl₃, wherein CF₂H and CF₃ are most preferred.

R² is selected from the group consisting of an optionally substituted C₁-C₈ alkyl group, an optionally substituted C₃-C₈ cycloalkyl group, an optionally substituted aryl group and an optionally substituted heteroaryl group. Preferred R² are ethyl, methyl and phenyl, wherein a methyl group is most preferred as R².

R³ is selected from the group consisting of H, X, COOR, OR, SR, C(O)NR₂, wherein R is selected from the group consisting of hydrogen, a C₁-C₁₂-alkyl, C₂-C₆ alkenyl, aryl, C₃-C₈ cycloalkyl, aralkyl or heteroaryl group, each of which is optionally substituted, with the proviso that both R in C(O)NR₂ may be the same or different, wherein X is a halogen atom and is selected from the group consisting of F, Cl, Br and I, and R is independently selected from the group consisting of hydrogen or a C₁-C₁₂-alkyl, C₂-C₆ alkenyl, aryl, cycloalkyl, aralkyl, heteroaryl, each of which can be optionally substituted. In a preferred aspect, R³ is H or X, wherein F is a preferred X. H is the most preferred R³.

R⁴ is selected from the group consisting of H, C₁-C₁₂-alkyl, C₂-C₆ alkenyl, C₃-C₈ cycloalkyl, aryl, heteroaryl or aralkyl group, each of which is optionally substituted. R⁴ is preferably selected from the group consisting of C₁-C₄ alkyl groups, H, optionally substituted phenyl and optionally substituted benzyl, wherein a methyl group is the most preferred group R⁴.

R⁵ is an -OH group,

R⁶ is selected from the group consisting of an optionally substituted C₁-C₈ alkyl group, an optionally substituted C₃-C₈ cycloalkyl group, an optionally substituted aryl group and an optionally substituted heteroaryl group. The optional substitution preferably is a substitution by at least one halogen selected from the group consisting of F, Cl, Br and I, wherein F and Cl are preferred. R⁶ defines the compound of formula (III). The compound of formula (III) can be liquid at ambient temperature and pressure, or solid at ambient temperature and pressure. In one preferred aspect, the compound of formula (III) is liquid at ambient temperature and pressure. Preferably, R⁶ is an optionally substituted C₁-C₈ alkyl group, more preferably, R⁶ is an optionally substituted methyl group. R⁶ thus, in a most preferable aspect, is selected from the group consisting of CH₃, CF₃, CF₂H, CCl₃ and CHCl₂. Compounds of formula (III) with R⁶ = CH₃ or CF₃ are most preferred. In another aspect, the compound of formula (III) is solid at ambient temperature and pressure. In these cases, R⁶ often is an optionally substituted aryl group, in particular an optionally substituted phenyl group. Phenyl groups substituted with one or more halogen atoms selected from F, Cl, Br and I are more preferred. For example, the compound of formula (III) can be selected from meta-chloroperoxybenzoic acid (mCPBA) and peroxybenzoic acid and its derivatives.

In one aspect, an auxiliary can be present in the step of oxidizing the compound of formula (I) into a compound of formula (II). The auxiliaries can enhance oxidation selectivity, reactivity and velocity of the reaction. The auxiliaries may act catalytically, or may be consumed by the reaction. The amount of auxiliaries can be, depending on their mode of action, stoichiometric, substoichiometric or superstoichiometric. Such auxiliaries can be, for example, BF₃ or salts such as triflates of rare earth compounds, carbonates, hydroxides, for example, talcites, and oxides, such as selenoxides.

The at least one catalyst, if present in the process of the present invention, often is present in an amount of from 0.01 to 30 mol% calculated on the amount of compound (II). Generally, the at least one catalyst in the process of the present invention is present in an amount of equal to or more than 0.01mol %, preferably equal to or more than 1 mol% calculated on the amount of compound (II). In some systems, an amount of equal to or more than 5 mol% calculated on the amount of compound (II) gives good results. Often, the at least one catalyst in the process of the present invention is present in an amount of equal to or less than 30 mol%, preferably equal to or less than 20 mol% calculated on the amount of compound (II). In some systems, an amount of equal to or less than 15 mol% calculated on the amount of compound (II) gives good results.

In one aspect of the present invention, at least one additional reagent can be present in the reaction, wherein the at least one additional reagent is a co-oxidant or an auxiliary. Co-oxidants can be, for example, TEMPO (2,2,6,6-Tetramethylpiperidinyloxyl), 4-hydroxy-TEMPO, 4-acetamido-TEMPO, 2-Azaadamantane N-Oxyl (AZADO), 9-Azabicyclo[3.3.1]nonane N-Oxyl (ABNO) and 9-Azanoradamantane N-oxyl (Nor-AZADO). Auxiliary agents can be, for example, KCl, N,N',N"-trihydroxyisocyanuric acid (THICA) and N-hydroxyphthalimide (NHPI), tetra-n-butylammonium bromide or even NaCl. Particularly suitable catalytic systems employing a co-oxidant and/or auxiliary are, for example, THICA/Mn(OAc)₂, NHPI/Mn(OAc)₂, NHPI/Mn(OAc)₂/Co(OAc)₂, THICA/Co(OAc)₂, THICA/Mn(OAc)₂/Co(OAc)₂, TEMPO/Fe(NO3)3˙9H₂O or TEMPO/Fe(NO3)3˙9H₂O/KCl.

In one aspect, the oxidation is carried out in at least one suitable solvent, such as dichloromethane, dichloroethane, methanol, ethanol, DMSO, DMF, acetonitrile or aliphatic ethers such as diethyl ether, or aromatic hydrocarbons such as toluene. Other very suitable solvents are carboxylic acids, in particular carboxylic acids with the formula R⁶COOH, preferably acetic acid. The presence of carboxylic acids with the formula R⁶COOH can catalyse the reaction. One preferred aspect concerns a process for the manufacture of a compound of formula (II) comprising a step of oxidizing a compound of formula (I) in the presence of at least one compound of formula (III) R⁶-C(O)O-OH, wherein the step of oxidizing a compound of formula (I) is performed in the presence of at least one carboxylic acid R⁶-COOH. More preferably, the compound of formula (III) R⁶-C(O)O-OH is applied as a 5-70, preferably 10-40 wt%, and more preferably 15-20 wt% solution which further comprises a compound of formula R⁶-COOH. In one aspect, the solution of compound of formula (III) also comprises water. Such solutions can be obtained, from example, commercially from the company Solvay, or can be obtained by reacting a compound of formula R⁶-COOH with hydrogen peroxide. The compound of formula (II) obtained is a compound with R²=OH which often is insoluble in the compound of formula R⁶-COOH, for example acetic acid or trifluoroacetic acid. After the reaction of oxidizing a compound of formula (I) to obtain a compound of formula (II) with R²=OH is performed, the compound of formula (II), often being insoluble in R⁶-COOH, can then be advantageously recovered by filtration, optionally washing and then drying. Washing preferably is performed with water and/or R⁶-COOH. Other techniques can be applied for isolation of the product, such as removal of volatiles, precipitation, distillation and crystallization. In one preferred aspect of the present invention, the recovered phase comprising the compound of formula R⁶-COOH, which may be a mixture of one or more compounds of formula R⁶-COOH, which can comprise the mother liquor and/or the washing liquor of the reaction, is recycled to a process according to the present invention, for example by partial oxidation of the recovered phase in order to obtain a mixture of compound R⁶-COOH and the compound of formula (III). Such reactions are known in the art, for example from WO08047263 and JP2015205845.

In one aspect, the process according to the present invention can comprise a step after the step in which the compound of formula (I) is oxidized in the presence of the at least one compound of formula (III) R⁶-C(O)O-OH, wherein the reaction mixture is treated with a peroxide scavenging agent, such as Na₂SO₃.

When the compound of formula (III) is an optionally substituted perbenzoic acid, in particular meta-chloroperoxybenzoic, the presence of halogenated aliphatic hydrocarbon solvents, such as dichloromethane, or aromatic solvents, such as toluene, is favourable, wherein the presence of halogenated aliphatic hydrocarbon solvents, such as dichloromethane, is preferred.

The oxidation often is carried out at temperatures of from 0°C to 120°C, preferably at temperatures of from 0°C to 50°C. The temperature is selected such as to take into consideration boiling and decomposition temperatures of reactants, products and solvents. Generally, the temperature in the step of oxidizing the compound of formula (I) in the process of the present invention is equal to or more than 0 °C, preferably equal to or more than 10°C. Often, the temperature in the step of oxidizing the compound of formula (I) in the process of the present invention is equal to or less than 50°C, preferably equal to or less than 40°C, and more preferably equal to or less than 30°C.

The invention also concerns a process as described for the manufacture of a compound of formula (II) which further comprises a step of reacting a compound of formula (IV) with a compound selected from the group consisting of compound (V), (VI) and (VII) to obtain the compound of formula (I) 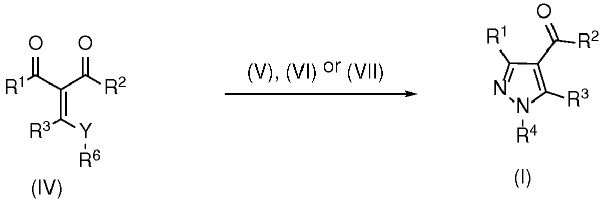 wherein compound (V), (VI) and (VII) are defined as follows: 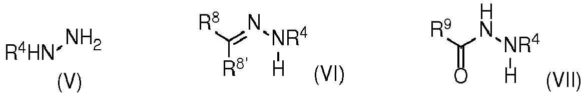
wherein Y is selected from the group consisting of S, O and NR⁷, wherein O and NR⁷ are preferred,
wherein R⁷ independently is selected from the group consisting of a C₁-C₁₂-alkyl, C₂-C₆ alkenyl or C₃-C₈-cycloalkyl group, each of which is optionally substituted
wherein R⁸ and R^{8'} independently from each other are selected from the group consisting of H, C₁-C₁₂-alkyl, C₂-C₆ alkenyl, cycloalkyl, aryl, heteroaryl or aralkyl group, each of which is optionally substituted,
R⁹ is selected from the group consisting of C₁₋C₁₂₋alkyl, OR¹⁰ and NR¹⁰R^{10'}, wherein R¹⁰ and R^{10'} independently are selected from the group consisting of C₁-C₁₂-alkyl and H,
and wherein R⁴ is defined as above.

Preferably, in such a process, Y is O and R⁷ is ethyl, or Y is NR⁷ and both R⁷ are methyl, R³ is H, R⁴ and R² are methyl, and R¹ is CF₂H or CF₃.

Often, R⁸ in (VI) is H, and R^{8'} is phenyl, benzyl or C₃ to C₆ cycloalkyl, each of which can be optionally substituted.

In one aspect, when Y = NR⁷, both R⁷ in (IV) with the N to which they are attached can form a heterocyclic substituent, such as a piperidinyl substituent.

Compounds of formula (IV) and their preparation are described, for example, in WO2017/054112. Compounds of formula (V), (VI) and (VII) and their preparation are described, for example, in WO2016/152886. The catalysts, oxidants, co-oxidants and auxiliary compounds are well known and can be purchased or made according to methods known to the person skilled in the art.

All embodiments of the present invention are particularly preferred when (I) is 1-(3-(difluoromethyl)-1-methyl-1H-pyrazol-4-yl)ethanone and (II) is 3-(difluoromethyl)-1-methyl-1H-pyrazole-4-carboxylic acid, and when (I) is 1-(3-(trifluoromethyl)-1-methyl-1H-pyrazol-4-yl)ethanone and (II) is 3-(trifluoromethyl)-1-methyl-1H-pyrazole-4-carboxylic acid.

The invention also concerns a process for the manufacture of an agrochemical or pharmaceutical compound, preferably a carboxamide SDHI fungicide, which comprises the process for manufacturing a compound of formula (II) as described above. SDHI fungicides are SDH inhibitors which target succinate dehydrogenase (SDH, so-called complex II in the mitochondrial respiration chain), and generally contain a carboxamide moiety, wherein the carbonyl carbon atom is attached to a pyrazole ring identical to the pyrazole system of formula (II).

An agrochemically or pharmaceutically active compound can, for example, be obtained by converting a compound of formula (II) obtained by the process according to the present invention into a carboxylic acid halide or anhydride, and reacting the carboxylic acid halide or anhydride with a primary or secondary amine to obtain a carboxamide which is an agrochemically or pharmaceutically active compound. Such reactions are known, for example, from WO2003070705, "Bioactive Heterocyclic Compound Classes", Ed. C. Lamberth und J. Dinges, Wiley 2012, S. 175-193 (Chapter 15, Pyrazole Carboxamide Fungicides Inhibiting Succinate Dehydrogenase) and "Modern Crop Protection Compounds", Ed. W. Krämer, U. Schirmer, P. Jeschke and M. Witschel, Wiley 2012, S. 627-639 and the literature cited in these books. In such a process for the manufacture of an agrochemical compound, for example compounds such as N-(3',4'-Dichlor-5-fluorbiphenyl-2-yl)-3-(difluormethyl)-1-methylpyrazol-4-carboxamid, 3-(difluoromethyl)-1-methyl-N-[2-(3',4',5'-trifluorophenyl)phenyl]pyrazole-4-carboxamide, N-(2-Bicyclopropyl-2-ylphenyl)-3-difluoromethyl-1-methyl-1H-pyrazol-4-carboxylic acid amide, 3-(Difluormethyl)-1-methyl-N-[1,2,3,4-tetrahydro-9-(1-methylethyl)-1,4-methanonaphthalen-5-yl]-1H-pyrazol-4-carboxamid or N-[(1RS,4SR)-9-(dichloromethylidene)-1,2,3,4-tetrahydro-1,4-methanonaphthalen-5-yl]-3-(difluoromethyl)-1-methyl-1H-pyrazole-4-carboxamide (and isomers) are obtained.

The new process according to the present invention allows for efficient syntheses of carboxylic acids or carboxylic acid derivatives, which are useful intermediates for, e.g., agrochemical and pharmaceutical compounds. Departing from easily accessible starting materials, such as methyl ketones, the carboxylic acids or carboxylic acid derivatives can be obtained while avoiding a high amount of salt waste which often also is difficult to dispose of and/or recycle due to organic impurities.

Should the disclosure of any patents, patent applications, and publications which are incorporated herein by reference conflict with the description of the present application to the extent that it may render a term unclear, the present description shall take precedence.

The following examples are intended to further explain the invention without limiting it.

All compounds can be obtained commercially or by methods known to the person skilled in the art. The manufacture of compounds of formula (I) is known, for example, from WO2016/152886, KR2017017372 and WO2017/064550.

### Example 1

0.43 g (2.5 mmol) of 1-(3-(difluoromethyl)-1-methyl-1H-pyrazol-4-yl)ethanone and 20 g (39 mmol) of peracetic acid (15 wt% in acetic acid) were mixed in a 50 mL three necked flask. The mixture was heated to 100°C overnight while being stirred. HPLC showed full conversion of 1-(3-(difluoromethyl)-1-methyl-1H-pyrazol-4-yl)ethanone into 3-(difluoromethyl)-1-methyl-1H-pyrazole-4-carboxylic acid.

### Example 2

30 g (58.5 mmol) of peracetic acid (15 wt% in acetic acid) is added to a 100 mL three necked flask. 0.65 g (3.75 mmol) of 1-(3-(difluoromethyl)-1-methyl-1H-pyrazol-4-yl)ethanone is added under stirring. The mixture is heated to 100°C overnight while being stirred. The mixture is cooled to 20°C, whereupon the product 3-(difluoromethyl)-1-methyl-1H-pyrazole-4-carboxylic acid precipitates. The precipitate is filtered, washed with water and dried to obtain the product.

### Example 3

The filtrate of example 2 is recycled into the process of example 2 by reaction with H₂O₂.

### Example 4

Hydrogen peroxide (90%) (1.2 g, 32 mmol) is mixed with 20 mL CHCl₃ at 0°C. Under stirring and cooling, 11.8 g (56 mmol) trifluoroacetic acid are added, and the mixture is stirred for 15 minutes at 0°C. 3.4 g (20 mmol) of 1-(3-(difluoromethyl)-1-methyl-1H-pyrazol-4-yl)ethanone in 20 mL CHCl₃ are added under stirring, the mixture is slowly heated to 70°C and maintained at this temperature for 5 hours. The mixture is cooled to 20°C, the volatiles removed in vacuum, and 40 mL of water are added under vigorous stirring. The product 3-(difluoromethyl)-1-methyl-1H-pyrazole-4-carboxylic acid precipitates. The precipitate is filtered, washed with water and dried to obtain the product.

### Example 5

Hydrogen peroxide (90%) (1.2 g, 32 mmol) is mixed with 20 mL CHCl₃ at 0°C. Under stirring and cooling, 11.8 g (56 mmol) trifluoroacetic acid are added, and the mixture is stirred for 15 minutes at 0°C. 3.8 g (20 mmol) of 1-(3-(trifluoromethyl)-1-methyl-1H-pyrazol-4-yl)ethanone in 20 mL CHCl₃ are added under stirring, the mixture is slowly heated to 70°C and maintained at this temperature for 5 hours. The mixture is cooled to 20°C, the volatiles removed in vacuum, the product 3-(trifluoromethyl)-1-methyl-1H-pyrazole-4-carboxylic acid washed with water and dried to obtain the product.

Examples 1 to 3 are repeated with 1-(3-(trifluoromethyl)-1-methyl-1H-pyrazol-4-yl)ethanoneto obtain 3-(trifluoromethyl)-1-methyl-1H-pyrazole-4-carboxylic acid.

## Claims

1. Process for the manufacture of a compound of formula (II) comprising a step of oxidizing a compound of formula (I) in the presence of at least one compound of formula (III) R⁶-C(O)O-OH 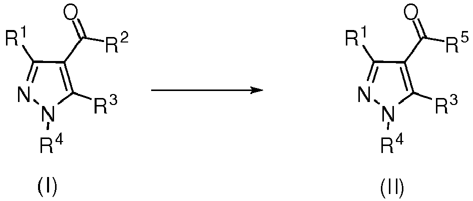 wherein
R¹ is selected from the group consisting of C₁-C₄-alkyl groups which is substituted by at least one halogen atom independently selected from the group consisting of F, Cl and Br or by a CF₃ group,
R² is selected from the group consisting of an optionally substituted C₁-C₈ alkyl group, an optionally substituted C₃-C₈ cycloalkyl group, an optionally substituted aryl group and an optionally substituted heteroaryl group, preferably R² is a methyl group
R³ is selected from the group consisting of H, X, COOR, OR, SR, C(O)NR₂, wherein R is selected from the group consisting of hydrogen, a C₁-C₁₂-alkyl, CN, C₂-C₆ alkenyl, aryl, C₃-C₈ cycloalkyl, aralkyl or heteroaryl group, each of which is optionally substituted, with the proviso that both R in C(O)NR₂ may be the same or different, wherein X is selected from the group consisting of F, Cl, Br and I, and R is independently selected from the group consisting of hydrogen or a C1-C12-alkyl group, C1-C12-alkyl, C2-C6 alkenyl, aryl, cycloalkyl, aralkyl, heteroaryl,
R⁴ is selected from the group consisting of H, C₁-C₁₂-alkyl, C₂-C₆ alkenyl, C₃-C₈ cycloalkyl, aryl, heteroaryl or aralkyl group, each of which is optionally substituted,
R⁵ is an -OH group,
and wherein R⁶ is selected from the group consisting of an optionally substituted C₁-C₈ alkyl group, an optionally substituted C₃-C₈ cycloalkyl group, an optionally substituted aryl group and an optionally substituted heteroaryl group.

2. Process according to claim 1, wherein R¹ is selected from the group consisting of CF₂H, CF₃, CClF₂, CCl₂H and CCl₃, wherein CF₂H and CF₃ are preferred.

3. Process according to claim 1 or 2, wherein R⁴ is a C₁-C₄ alkyl group which is optionally substituted, preferably a methyl group.

4. Process according to anyone of claims 1 to 3, wherein R³ is H or X, wherein X is selected from the group consisting of F, Cl, Br and I, preferably R³ is H.

5. Process according to anyone of claims 1 to 4, wherein R⁶ is an optionally substituted C₁-C₈ alkyl group, more preferably, R⁶ is an optionally substituted methyl group.

6. Process according to claim 5, wherein R⁶ is selected from the group consisting of CH₃, CF₃, CF₂H, CCl₃ and CHCl₂.

7. Process according to anyone of claims 1 to 4, wherein R⁶ is an optionally substituted aryl group, more preferably, R⁶ is an optionally substituted phenyl group.

8. Process according to claim 7, wherein the compound of formula (III) is an optionally substituted peroxobenzoic acid, wherein meta-chloroperoxybenzoic acid is preferred.

9. Process according to anyone of claims 1 to 8, wherein the step of oxidizing a compound of formula (I) is performed in the presence of at least one carboxylic acid R⁶-COOH.

10. Process according to anyone of claims 1 to 9, wherein the step of oxidizing a compound of formula (I) is carried out at temperatures of from 0°C to 120°C.

11. Process according anyone of claims 1 to 10, wherein the at least one catalyst is present in an amount of from 0.01 to 30 mol% calculated on the amount of compound (II).

12. Process according anyone of claims 1 to 11, wherein at least one additional reagent is present in the reaction, wherein the at least one additional reagent is a co-oxidant or an auxiliary.

13. Process according anyone of claims 1 to 12, wherein the process for the manufacture of a compound of formula (II) further comprises a step of reacting a compound of formula (IV) with a compound selected from the group consisting of compound (V), (VI) and (VII) to obtain the compound of formula (I) 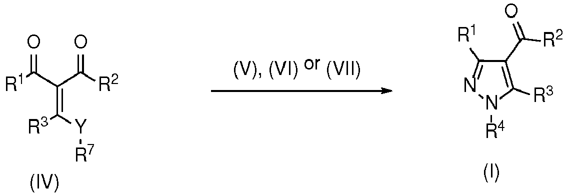 wherein compound (V), (VI) and (VII) are defined as follows: 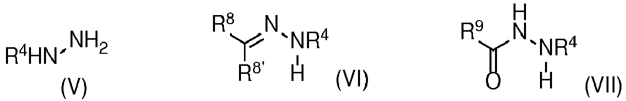
wherein Y is selected from the group consisting of S, O and NR⁷, wherein O and NR⁷ are preferred,
wherein R⁷ independently is selected from the group consisting of a C₁-C₁₂-alkyl, C₂-C₆ alkenyl or C₃-C₈-cycloalkyl group, each of which is optionally substituted
wherein R⁸ and R^{8'} independently from each other are selected from the group consisting of H, C₁-C₁₂-alkyl, C₂-C₆ alkenyl, cycloalkyl, aryl, heteroaryl or aralkyl group, each of which is optionally substituted,
R⁹ is selected from the group consisting of C₁-C₁₂-alkyl, OR¹⁰ and NR¹⁰R^{10'}, wherein R¹⁰ and R^{10'} independently are selected from the group consisting of C₁-C₁₂-alkyl and H.

14. Process according to claim 13, wherein Y is O and R⁷ is ethyl, or Y is NR⁷ and both R⁷ are methyl,
R³ is H,
R⁴ and R² are methyl,
and R¹ is CF₂H or CF₃.

15. Process for the manufacture of an agrochemical or pharmaceutical compound, preferably a carboxamide fungicide, which comprises the process according to anyone of claims 1 to 14.
